(19) Europäisches Patentamt
European Patent Office
Office européen des brevets

(11) **EP 2 523 697 B1**

(12) **FASCICULE DE BREVET EUROPEEN**

(45) Date de publication et mention de la délivrance du brevet:
**03.12.2014 Bulletin 2014/49**

(21) Numéro de dépôt: **11704285.3**

(22) Date de dépôt: **13.01.2011**

(51) Int Cl.:
***A61L 15/58*** *(2006.01)*

(86) Numéro de dépôt international:
**PCT/FR2011/050062**

(87) Numéro de publication internationale:
**WO 2011/086329 (21.07.2011 Gazette 2011/29)**

(54) **NOUVEAU PANSEMENT COMPRENANT DES AGGLOMERATS DE PARTICULES CAPABLES DE GELIFIER OU SE SOLUBILISER RAPIDEMENT.**

NEUE BANDAGE MIT AGGLOMERATEN VON PARTIKELN ZUR SCHNELLEN GELIERUNG ODER AUFLÖSUNG

NOVEL BANDAGE INCLUDING AGGLOMERATES OF PARTICLES SUITABLE FOR RAPIDLY GELLING OR SOLUBILISING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.01.2010 FR 1050221**

(43) Date de publication de la demande:
**21.11.2012 Bulletin 2012/47**

(73) Titulaire: **Laboratoires Urgo**
**21300 Chenove (FR)**

(72) Inventeur: **PERNOT, Jean-Marc**
**F-21000 Dijon (FR)**

(74) Mandataire: **Hubert, Philippe**
**Cabinet Beau de Loménie**
**158, rue de l'Université**
**75340 Paris Cedex 07 (FR)**

(56) Documents cités:
**EP-A1- 0 621 042      EP-A2- 1 020 198**
**WO-A1-99/27975      US-A- 5 681 305**

**Description**

La présente invention a pour objet un nouveau pansement repositionnable comprenant une masse hydrocolloïde adhésive destiné au traitement des plaies telles que les plaies exudatives, les brûlures, les lésions dermo-épidermiques superficielles, profondes, chroniques ou aïgues et en particulier au traitement de l'ampoule.

Les pansements comprenant des hydrocolloïdes sont connus depuis plus de 20 ans. Ils sont constitués d'un support sur lequel est déposée une masse adhésive comprenant des hydrocolloïdes. On peut citer, à titre d'exemples, les produits commercialisés sous les dénominations Algoplaque® par les Laboratoires URGO et Comfeel® par la société Coloplast. Des pansements comprenant une masse adhésive comprenant des hydrocolloïdes, spécifiquement destinés au traitement de l'ampoule, sont également connus et commercialisés par exemple sous les dénominations Urgo Traitement Ampoules® par les Laboratoires URGO et Compeed® par la société Johnson & Johnson.

Pour permettre une bonne absorption des exsudats de la plaie, ces pansements contiennent des quantités relativement importantes (de l'ordre de 20 à 50 % en masse) d'hydrocolloïdes. De manière préférentielle, ces pansements sont conçus pour tenir en place sans l'aide d'une bande adhésive complémentaire, en adhérant directement à la peau.

La masse adhésive de ces pansements connus est habituellement constituée d'une phase continue hydrophobe dans laquelle est dispersée une phase discontinue de particules d'hydrocolloïdes destinées à absorber les exsudats de la plaie.

L'absorption des exsudats par les hydrocolloïdes provoque la gélification de la masse adhésive, ce qui permet de retirer sans douleur le pansement de la plaie après son utilisation.

Pour assurer le maintien dans le temps de leur capacité d'absorption et de leur cohésion lors du retrait, ces pansements possèdent un pouvoir adhésif initial important. Ceci est encore plus vrai pour les pansements destinés au traitement des ampoules qui doivent être positionnés dans des zones courbes ou difficiles d'accès et qui sont soumis à de fortes contraintes mécaniques lors de leur utilisation.

Pour augmenter le pouvoir adhésif de ces pansements, on a travaillé :

- d'une part sur la composition qualitative et quantitative des masses adhésives les constituant; et
- d'autre part sur la forme de ces pansements, notamment en leur donnant des bords biseautés.

De tels pansements et leurs compositions sont bien connus et décrits par exemple dans les documents EP 264 299, EP 503 029, EP 1 020 198 et FR 2 495 473.

Toutefois, en raison de leur pouvoir adhésif important, ces pansements ne peuvent être repositionnés aisément au moment de leur mise en place sur la peau du patient. En effet, le retrait de ces pansements est très douloureux car le pansement adhère à la plaie ou à l'ampoule, tant qu'il n'a pas absorbé les exsudats ce qui peut nécessiter un temps relativement long de l'ordre de 15 minutes ou plus.

Le repositionnement du pansement, lors de sa mise en place, est cependant très souvent nécessaire, par exemple lorsque la surface du corps sur laquelle ce pansement doit être appliqué n'est pas plane, et ce problème est bien connu des personnels soignants.

En outre, ces pansements sont d'autant plus difficiles à mettre en place qu'ils sont généralement très fins, et, dans le cas des pansements destinés au traitement de l'ampoule, de petites dimensions et qu'ils doivent être positionnés dans des endroits difficiles d'accès : orteils, voûtes plantaires, talons, etc.

De nombreux utilisateurs ont déjà été confrontés à ce problème de repositionnement, en particulier dans le cas des pansements destinés au traitement de l'ampoule que l'on souhaite également pouvoir repositionner pour des raisons de discrétion et d'esthétique, par exemple pour éviter la formation de plis ou pour le dissimuler derrière la lanière d'une chaussure.

Si de nombreux systèmes applicateurs ont été développés depuis vingt ans pour faciliter la pose de ces pansements, ces systèmes ne permettent cependant pas de résoudre de manière satisfaisante le problème du repositionnement, et en particulier il n'existe pas à ce jour de solution idéale au problème du retrait sans douleur d'un pansement adhésif immédiatement après une première mise en place.

Le document EP 0 621 042 décrit un pansement hydrocolloïde dont la couche adhésive comprend un réseau tridimensionnel constitué de filaments polymériques revêtus d'une matière adhésive insoluble dans l'eau et les reliant entre eux à l'intérieur duquel sont dispersés des hydrocolloïdes. Ce réseau polymérique est destiné à maintenir l'intégrité et la cohésion du pansement même lorsque celui-ci a absorbé une quantité importante de liquide.

Le problème du retrait sans douleur du pansement immédiatement après sa pose, afin de permettre son repositionnement, tout en conservant ses propriétés d'adhésion, de cohésion et d'absorption au cours du temps, n'est d'aucune façon évoqué dans ce document antérieur pas plus que n'est évoquée l'utilisation de polymères résorbables et solubles spécifique.

Dans ces conditions, la présente invention a pour but de résoudre le nouveau problème technique consistant en la fourniture d'un pansement hydrocolloïde adhésif d'une nouvelle conception, qui puisse être retiré sans douleur immédiatement après sa pose pour être repositionné une ou plusieurs fois, tout en conservant ses propriétés d'adhésion, de cohésion et d'absorption au cours du temps.

Il a été découvert, et ceci constitue le fondement de la présente invention, qu'il était possible de résoudre ce problème technique, d'une façon relativement simple et utilisable à l'échelle industrielle, en modifiant la surface destinée à venir au contact de la peau d'un pansement adhésif hydrocolloïde traditionnel en y déposant des agglomérats de particules préconstitués aptes à se solubiliser ou gélifier au contact des exsudats de la plaie sensiblement totalement et en une durée très courte de préférence inférieure à 10 secondes, et de préférence encore inférieure à 1 seconde.

Ainsi, selon un premier aspect, la présente invention a pour objet un pansement comprenant une masse adhésive comprenant des hydrocolloïdes caractérisé en ce qu'il comprend, sur une partie au moins de la surface de la masse adhésive destinée à venir au contact de la plaie en position d'utilisation, une pluralité d'agglomérats constitués de particules liés entre elles, de préférence selon une configuration multicouches, lesdites particules étant constituées pour 90 % au moins , et de préférence 95 % au moins d'entre elles d'un (ou plusieurs) matériau(x) :

- choisi(s) parmi

  - les polysaccharides et en particulier l'amidon, les amidons modifiés, la maltodextrine, les gommes comme en particulier la gomme arabique, la gomme xanthane ou la gomme d'acacia, le pullulan, les dextranes, la cellulose, les dérivés cellulosiques comme en particulier la méthylcellulose, l'hydroxypropylméthylcellulose, l'hydroxypropylcellulose, l'hydroxyéthylcellulose, les sucres, la pectine, les alginates, les carraghénanes, l'agar-agar ;
  - les protéines et en particulier la gélatine, l'albumine, le collagène et la caséine ;
  - les polymères synthétiques superabsorbants et en particulier les polyacrylates ;
  - les polymères synthétiques hydrosolubles et en particulier l'alcool polyvinylique ;

  et les mélanges de ces composés ; et
- apte(s) à se solubiliser ou gélifier en moins de 10 secondes et de préférence encore en moins de 1 seconde au contact des exsudats de la plaie.

Comme on le comprend, en se solubilisant ou en gélifiant rapidement au contact des exsudats de la plaie, les agglomérats de particules précités vont former une couche intermédiaire non adhérente à la plaie immédiatement (quelques secondes au plus) après la première mise en place du pansement en permettant ainsi, en cas de besoin, de retirer le pansement de la plaie sans douleur et de le repositionner.

Il a été constaté que malgré la présence de ces agglomérats de particules hydrosolubles ou gélifiables, le pansement conserve le même profil d'adhésion au retrait, les mêmes propriétés d'absorption qu'un pansement de même constitution mais ne comprenant pas ces agglomérats. En particulier, il n'a pas été observé, avec ces nouveaux pansements, de migration des exsudats au-delà ou autour de la partie de la surface de la masse adhésive recouverte par la couche formée après gélification ou solubilisation des agglomérats qui aurait pu altérer les propriétés adhésives ou les propriétés d'absorption et de cohésion du pansement en créant un différentiel d'absorption entre ces deux zones.

Selon une caractéristique particulière, ces agglomérats peuvent contenir des particules de substances actives qui seront rapidement relarguées et qui faciliteront la cicatrisation de l'ampoule ou de la plaie.

Le pansement selon la présente invention peut être obtenu aisément, par simple dépose des agglomérats de particules, préalablement formés, sur la surface destinée à venir au contact de la plaie d'un pansement adhésif contenant des hydrocolloïdes.

Les masses adhésives contenant des hydrocolloïdes susceptibles d'être utilisées pour la fabrication du pansement conforme à l'invention sont celles habituellement utilisées par l'homme de l'art pour la fabrication de pansements adhésifs hydrocolloïdes.

D'une façon générale, les masses adhésives des pansements selon l'invention présentent une adhésivité d'au moins 150 cN/ cm, et de préférence comprise entre 200 cN/cm et 8 N/cm mesurée sur plaque d'acier selon la norme européenne EN 1939 (mesure à 90°, vitesse de 100 mm/ min).

D'une façon générale, ces masses adhésives seront constituées d'une matrice élastomérique contenant des particules hydrocolloïdes et un (ou plusieurs) composé(s) destiné(s) à conférer des propriétés d'adhérence à ladite masse connu(s) sous le nom de « tackifiant(s) ».

Par « matrice élastomérique », on entend désigner ici une composition comprenant un (ou plusieurs) élastomère(s) choisi(s) parmi les copolymères séquencés poly(styrène-oléfine-styrène), dont les séquences oléfines peuvent être constituées d'unités isoprène, butadiène, éthylène-butylène, éthylène-propylène et leurs mélanges.

Dans le cadre de la présente invention, on préférera les copolymères triblocs poly(styrène-isoprène-styrène) (en abrégé : poly(SIS)) et les mélanges de copolymères triblocs poly(SIS) et de copolymères diblocs poly(styrène-isoprène), et en particulier les poly(SIS) ayant une teneur en styrène comprise entre 14 et 52 % et de préférence entre 14 et 30 % en poids rapporté au poids dudit poly(SIS).

De tels produits bien connus de l'homme de l'art sont par exemple commercialisés par la société Kraton sous la dénomination KRATON®D ou par la société Dexco Polymers LP sous la dénomination VECTOR®.

Parmi les copolymères triblocs poly(SIS) préférés, on peut citer en particulier les produits commercialisés sous les dénominations KRATON®D-1111CS, KRATON®D-1107 ou KRATON®1161, VECTOR®4114 et VECTOR®4113.

Des copolymères triblocs poly(styrène-butadiène-styrène) peuvent également être utilisés dans le cadre de l'invention.

Parmi ces copolymères poly(styrène-butadiène-styrène), on peut citer en particulier le produit commercialisé sous la dénomination KRATON®D-1102 par la société Kraton.

De préférence, les élastomères formant la matrice élastomérique seront présents, au sein de la masse adhésive des pansements selon l'invention en une quantité de 10 à 30 % en poids, et de préférence de 15 à 25 % en poids, du poids total de la masse adhésive.

D'une façon générale, la matrice élastomérique précitée incorpore un (ou plusieurs) hydrocolloïde(s).

Par « hydrocolloïde» on entend désigner ici tout composé habituellement utilisé par l'homme de l'art pour son aptitude à absorber les liquides hydrophiles tels que l'eau, le sérum physiologique ou les exsudats d'une plaie.

A titre d'exemple d'hydrocolloïde susceptible d'être utilisé dans le cadre de l'invention, on peut citer la pectine, les alginates, les gommes végétales naturelles comme en particulier la gomme de Karaya, les dérivés de cellulose comme en particulier les carboxyméthylcelluloses et leurs sels de métal alcalin, notamment de sodium ou de calcium, ainsi que les polymères synthétiques à base de sels de l'acide acrylique, connus sous l'appellation "superabsorbants", comme par en particulier les produits commercialisés par la société BASF sous la dénomination LUQUASORB® 1003 ou par la société CIBA Specialty chemicals sous la dénomination SALCARE® SC91. Bien entendu, des mélanges de ces produits peuvent être utilisés à titre d'hydrocolloïdes.

Il est à noter que ces composés sont présents au sein de la masse élastomérique et bien que susceptibles de se gélifier au contact des exsudats, leur éventuelle gélification n'est susceptible de se produire qu'après un lapse de temps relativement long, pouvant atteindre plusieurs heures, de sorte que ces composés ne sont pas susceptibles de résoudre le problème du repositionnement du pansement.

Les hydrocolloïdes préférés dans le cadre de la présente invention sont les sels de métal alcalin de la carboxyméthylcellulose, et en particulier la carboxyméthylcellulose de sodium.

La quantité d'hydrocolloide(s) incorporée dans la matrice élastomérique sera adaptée en fonction du niveau d'absorption souhaité. D'une façon générale, la quantité d'hydrocolloïde(s) pourra être de l'ordre de 2 à 50 % en poids, rapportée au poids total de la masse adhésive.

Dans le cadre de la présente invention, on utilisera de préférence une quantité d'hydrocolloïde(s) comprise entre 20 et 50 % en poids rapportée au poids total de la masse adhésive si l'on désire réaliser un pansement absorbant du type de ceux décrits dans les documents EP 1 061 965, EP 1 165 717 et EP 0 927 051.

La matrice élastomérique contenant des particules hydrocolloïdes est rendue adhésive par l'ajout de produits dits « tackifiants » tels que ceux qui sont habituellement utilisés par l'homme de l'art dans la préparation des adhésifs sensibles à la pression à base d'élastomères et en particulier de copolymères séquencés poly(styrène-oléfine-styrène). Pour une description détaillée de ces produits, on pourra se reporter aux documents de l'état de la technique mentionnés précédemment ou à l'ouvrage de Donatas Satas "Handbook of Pressure Sensitive Technology", 3rd Edition, 1999, pages 346 à 398.

D'une façon générale, on pourra utiliser un (ou plusieurs) produit(s) tackifiant(s) qui sera (seront) incorporés(s) à la masse hydrocolloïde dans une large proportion de l'ordre de 1 à 70 % en poids rapportée au poids total de la masse adhésive, qui sera déterminée en fonction de la nature et de la proportion relative des autres constituants de cette dernière, pour obtenir le pouvoir adhésif souhaité pour la composition finale.

De préférence, le(s) produit(s) tackifiant(s) représentera (représenteront) de 10 à 40 % en poids, du poids total de la masse hydrocolloïde.

Les produits tackifiants susceptibles d'être utilisés dans le cadre de la présente invention pourront être choisis parmi les résines tackifiantes, les polyisobutylènes de bas poids moléculaire, les polybutènes de bas poids moléculaire ou leurs mélanges.

Parmi les résines tackifiantes susceptibles d'être utilisées selon l'invention, on peut mentionner les résines polyterpènes ou terpènes modifiées, les résines de colophane, les résines hydrocarbonnées, les mélanges de résine cyclique, aromatique et aliphatique, etc., ou des mélanges de ces résines.

De tels produits sont commercialisés par exemple :

- par la société GOODYEAR sous la dénomination WINGTACK®, comme en particulier la résine de synthèse formée de copolymères en C5/C9 (WINGTACK® 86) ou la résine à base de polyterpène synthétique (WINGTACK® 10) ;
- ou encore par la société HERCULES sous la dénomination KRISTALEX® comme en particulier la résine à base d'alpha-méthylstyrène (KRISTALEX® 3085).

Ces résines tackifiantes peuvent être utilisées seules ou en mélange avec d'autres tackifiants, de préférence dans une proportion de 20 à 50 % en poids, et plus particulièrement de 25 à 35 % en poids, rapportée au poids total de la masse adhésive.

Parmi les polybutènes de bas poids moléculaire susceptibles d'être utilisés selon l'invention, on peut citer par exemple les produits commercialisés par la société BP CHIMIE sous la dénomination NAPVIS® et en particulier le produit commercialisé sous la dénomination NAPVIS® 10.

Ces polybutènes peuvent être utilisés seuls ou en mélange avec d'autres tackifiants, de préférence dans une proportion de 5 à 30 % en poids, et plus particulièrement de 8 à 15 % en poids, rapportée au poids total de la masse adhésive.

Parmi les polyisobutylènes de bas poids moléculaire susceptibles d'être utilisés selon l'invention, on peut citer les polyisobutylènes ayant un poids moléculaire de l'ordre de 40 000 à 80 000 daltons, comme par exemple les produits commercialisés par la société BASF sous la dénomination OPPANOL® et en particulier les produits commercialisés sous les dénominations OPPANOL®B12 et OPPANOL®B15.

Ces polyisobutylènes pourront être utilisés seuls ou en mélange avec d'autres tackifiants, de préférence dans une proportion de 5 à 30 % en poids, et plus particulièrement de 8 à 15 % en poids, rapportée au poids total de la masse adhésive.

Divers composés additionnels pourront être ajoutés à la matrice élastomérique contenant les composés tackifiants et hydrocolloïdes précités pour obtenir des masses adhésives hydrocolloïdes qui présentent des propriétés d'élasticité, d'adhésion, de stabilité dans le temps et de cohésion optimisées.

De tels composés sont par exemple des stabilisants comme en particulier des antioxydants, des plastifiants comme en particulier les polybutènes ou les huiles plastifiantes, ou des agents permettant d'améliorer la cohésion comme en particulier des caoutchoucs butyle ou des polyisobutylènes de haut poids moléculaire.

Parmi les polyisobutylènes de haut poids moléculaire susceptibles d'être utilisés selon l'invention pour améliorer la cohésion de la masse adhésive, on peut citer les polyisobutylène ayant un poids moléculaire de l'ordre de 400 000 à 2 000 000 daltons, comme par exemple les produits commercialisés par la société BASF sous les dénominations Oppanol®B12 SFN ou Oppanol®B30 SF.

Ces polyisobutylènes de haut poids moléculaire pourront être utilisés seuls ou en mélange, de préférence dans une proportion de 2 à 20 % en poids, et plus particulièrement de 5 à 15 % en poids, rapportée au poids total de la masse adhésive.

Par « stabilisant » on entend désigner ici tout composé apte à assurer la stabilité vis-à-vis de l'oxygène (antioxydant), la chaleur, l'ozone et les rayonnements ultra violets des composés utilisés dans la formulation des masses hydrocolloïdes adhésives, en particulier des résines tackifiantes et des copolymères séquencés. Ces composés stabilisants sont bien connus et pourront être utilisés seuls ou en mélange.

Parmi les composés antioxydants susceptibles d'être utilisés selon l'invention, on peut citer les antioxydants phénoliques, comme par exemple les produits commercialisés par la société CIBA-GEIGY sous les dénominations IRGANOX®1010, IRGANOX®565 et IRGANOX® 1076 ainsi que les antioxydants soufrés, comme par exemple le dibutyldithiocarbamate de zinc commercialisé par la société AKZO sous la dénomination PERKACIT®ZDBC.

Ces composés peuvent être utilisés seuls ou en mélange, de préférence dans une proportion de 0 à 2 % en poids, et plus particulièrement de 0,1 à 0,6 % en poids, rapportée au poids total de la masse hydrocolloïde.

Dans le cadre de la présente invention, on utilisera de façon préférée l'association de l'IRGANOX®1010 et du PERKACIT®ZDBC.

Parmi les composés plastifiants susceptibles d'être utilisés selon l'invention, on peut citer les plastifiants habituellement utilisés par l'homme de l'art pour la préparation de masses adhésives hydrocolloïdes et en particulier les polybutènes comme par exemple les produits commercialisés par la société BP CHEMICALS sous la dénomination NAPVIS® 10, les huiles plastifiantes ou encore des dérivés de phtalate tels que le dioctylphtalate.

L'utilisation des huiles plastifiantes est particulièrement préférée dans le cadre de la présente invention.

Par « huile plastifiante » on entend désigner ici les huiles minérales ou végétales couramment employées par l'homme de l'art pour plastifier les copolymères séquencés du type styrène-oléfine-styrène utilisés dans la composition des masses adhésives hydrocolloïdes.

Ces huiles minérales sont généralement constituées de mélanges dans des proportions variables de composés de nature paraffinique, naphténique ou aromatique.

Parmi les huiles plastifiantes susceptibles d'être utilisées selon l'invention, on peut citer les produits commercialisés par la société SHELL sous les dénominations ONDINA® et RISELLA® qui sont constitués de mélanges à base de composés naphténiques et paraffiniques ou sous la dénomination CATENEX® qui sont constitués de mélanges à base de composés naphténiques, aromatiques et paraffiniques.

Dans le cadre de la présente invention, on utilisera de façon préférentielle l'huile plastifiante minérale commercialisée sous la dénomination ONDINA®68.

Ces composés plastifiants peuvent être utilisés seuls ou en mélange, de préférence dans une proportion de 5 à 20 % en poids, et plus particulièrement de 7 à 15 % en poids, rapportée au poids total de la masse hydrocolloïde.

La masse adhésive hydrocolloïde des pansements selon l'invention peut encore comprendre un ou plusieurs composé(s) tensioactif(s) en une quantité inférieure ou égale à 10 % en poids, de préférence inférieure ou égale à 5 % en poids, rapportée au poids total de la masse hydrocolloïde.

Un composé tensioactif préféré dans le cadre de la présente invention est le composé commercialisé sous la dénomination AcResin®.

Un autre composé tensioactif préféré dans le cadre de la présente invention est le polysorbate 80, comme par exemple le produit commercialisé par la société SEPPIC sous la dénomination MONTANOX® 80.

Dans le cadre de la présente description, par l'expression « agglomérat de particules » on entend désigner un ensemble de particules de petites tailles, c'est à dire ayant une dimension moyenne comprise entre 500 nm et 1000 $\mu$m, de préférence comprise entre 1 $\mu$m et 500 $\mu$m, liées entre elles, de préférence selon une disposition multicouches, par exemple en forme de framboise.

Chaque agglomérat a généralement une dimension moyenne comprise entre 5 et 2 000 $\mu$m et sera donc constitué d'environ 10 à 10 000 particules, de préférence d'environ 100 à 1000 particules.

De tels agglomérats de particules peuvent être préparés, de façon connue en soi par des procédés de granulation en voie humide comme en particulier par spray drying ou dans un lit d'air fluidisé en utilisant généralement l'eau ou un mélange aqueux comme liant.

D'une façon générale, les particules formant les agglomérats sont constituées majoritairement, c'est-à-dire pour 90 % au moins, et de préférence pour 95 % au moins d'entre elles, d'un matériau choisi parmi les polysaccharides, les protéines et des polymères synthétiques, et apte à se solubiliser ou gélifier en moins de 10 secondes et de préférence encore en moins de 1 seconde au contact des exsudats de la plaie.

Par l'expression « matériau apte à se solubiliser» on entend désigner tout matériau capable de se dissoudre dans les exsudats de la plaie en formant une solution homogène.

Par l'expression « matériau apte à gélifier » on entend désigner tout matériau capable de former un gel au contact des exsudats de la plaie, ie une substance présentant une viscosité supérieure à celle de l'eau, par exemple supérieure à $10^{-3}$ Pa.s et de préférence supérieure à $10^{-2}$ Pa.s.

Parmi les polysaccharides susceptibles d'être utilisés selon l'invention, on peut citer l'amidon, les amidons modifiés, la maltodextrine, les gommes comme en particulier la gomme arabique, la gomme xanthane ou la gomme d'acacia, le pullulan, les dextranes, la cellulose, les dérivés cellulosiques comme en particulier la méthylcellulose, l'hydroxypropylméthylcellulose, l'hydroxypropylcellulose, l'hydroxyéthylcellulose, les sucres, la pectine, les alginates, les carraghénanes, l'agar-agar.

D'excellents résultats ont été obtenus en utilisant des particules de maltodextrine qui constitue donc un matériau préféré pour la mise en oeuvre de l'invention.

Parmi les protéines susceptibles d'être utilisées selon l'invention, on peut citer la gélatine, l'albumine, le collagène et la caséine.

Parmi les polymères synthétiques susceptibles d'être utilisés selon l'invention, on peut citer les polymères superabsorbants comme en particulier les polyacrylates ainsi que les polymères synthétiques hydrosolubles comme en particulier l'alcool polyvinylique.

Les agglomérats utilisés dans le cadre de la présente invention peuvent être constitués de particules de différentes natures, en fonction du degré de solubilité, de la résistance mécanique ou de la résistance à la température désirés.

Selon un mode de réalisation particulier de l'invention, les agglomérats contiennent une (ou plusieurs) substance(s) active(s).

Dans le cas où une pluralité de substances actives est utilisée, celles-ci pourront être incorporées soit individuellement dans des agglomérats séparés, soit sous forme de mélange au sein d'un même agglomérat.

Les substances actives susceptibles d'être utilisées dans le cadre de la présente invention peuvent être choisies parmi les antifongiques, les anti-microbiens ou antibactériens tels que la sulfadiazine argentique, les régulateurs de pH, les accélérateurs de cicatrisation tels que l'acide hyaluronique, les vitamines, les agents hydratants, les oligo-éléments, les anesthésiques locaux, les piégeurs d'odeur, le menthol, le salicylate de méthyle, les hormones, les anti-inflammatoires et les mélanges de ces composés.

Une substance active préférée dans le cadre de l'invention est l'acide hyaluronique.

Selon l'invention les agglomérats sont disposés, de façon uniforme ou non, à la surface de la masse adhésive hydrocolloïde destinée à venir en contact avec la plaie. En d'autres termes, ces agglomérats ne seront pas présents à l'intérieur de la masse adhésive hydrocolloïde.

D'une façon générale, les agglomérats recouvriront entre 5 et 90 %, et de préférence entre 10 et 60 %, de la surface de la masse adhésive venant au contact de la plaie, selon l'application envisagée. Ainsi, le taux de recouvrement de la surface de la masse adhésive venant au contact de la plaie sera de l'ordre de 10 % à 50 % pour un pansement destiné au traitement de l'ampoule et de l'ordre de 20 % à 70 % pour un pansement destiné au traitement de l'escarre ou de l'ulcère.

Ces agglomérats pourront être incorporés au pansement conforme à l'invention par tout procédé permettant leur dépôt à la surface de la masse adhésive hydrocolloïde.

Par exemple, cette opération peut être avantageusement réalisée par saupoudrage des agglomérats sur la surface de la masse adhésive hydrocolloïde au moyen d'un distributeur de poudre connu de l'homme du métier.

Selon un mode de réalisation préféré de l'invention, les agglomérats préalablement formés seront disposés uniquement au niveau de la zone du pansement destinée à venir en contact avec la zone de la plaie à traiter.

Par exemple, les agglomérats pourront être déposés au niveau des zones destinées à constituer la partie centrale des pansements ou répartis de façon régulière ou non à la surface de ces zones.

Les agglomérats pourront être déposés selon une forme géométrique quelconque, par exemple une forme carrée, rectangulaire, ovale ou striée. A cet effet, l'opération de dépôt peut être réalisée au moyen d'un pochoir permettant d'obtenir la forme désirée.

La quantité d'agglomérats déposée peut varier dans de larges proportions et sera généralement comprise entre 5 et 500 grammes par mètre carré de pansement, de préférence comprise entre 10 et 150 g/m$^2$.

Les pansements de l'invention peuvent être de différents types mais comprendront de préférence un support.

La masse adhésive portant les d'agglomérats peut être complexée à un support du type de ceux utilisés dans les pansements commercialisés par la société Laboratoires Urgo sous les dénominations Algoplaque®, ou par la société Convatec sous la dénomination Duoderm® ou encore par la société Coloplast sous la dénomination Comfeel®. De tels pansements sont décrits dans les documents FR 2 392 076, FR 2 495 473, WO 98/10801 et EP 264 299.

D'une façon générale, le choix du support sera réalisé en fonction des propriétés requises (étanchéité, élasticité, etc.) dans l'application recherchée.

Ainsi, le pansement selon l'invention peut comprendre un support tel qu'un film formé d'une ou plusieurs couches et d'une épaisseur variable de 5 à 150 $\mu$m ; un non-tissé ou encore une mousse ayant une épaisseur de 10 à 500 $\mu$m sur lequel la masse adhésive hydrocolloïde a été enduite, de façon continue ou non continue.

Ces supports à base de matériaux synthétiques ou naturels sont bien connus de l'homme de l'art.

Parmi les supports en forme de mousse utilisables dans le cadre de l'invention, on peut ainsi citer les mousses en polyéthylène, en polyuréthane, en PVC par exemple.

Parmi les supports non-tissés utilisables dans le cadre de l'invention, on peut citer les non-tissés en polypropylène, polyéthylène, polyuréthane, polyamide, ou polyester par exemple.

Dans le cadre de la présente invention, on préférera utiliser des supports en forme de films, et notamment des films en polyuréthanne comme par exemple les films commercialisés par la société Smith et Nephew sous la référence LASSO® réalisés à partir du polyuréthanne commercialisé par la société BF GOODRICH sous la dénomination ESTANE® ; des films en polyéthylène à basse densité comme par exemple les films commercialisés par la société SOPAL ; des films à base de copolymère thermoplastique de polyéther-polyester comme par exemple les produits commercialisés par la société DUPONT DE NEMOURS sous la dénomination Hytrel® ; ou encore des films complexes associant un film de polyuréthanne et un non-tissé.

Selon une variante de réalisation de l'invention, le pansement peut comporter une couche absorbante disposée entre le support et la masse hydrocolloïde adhésive. Cette couche absorbante peut être constituée de tout type de matériau absorbant tel que par exemple une mousse (comme en particulier une mousse polyuréthane), un non-tissé, une couche de polymère super absorbant, ou une combinaison de ces matériaux.

Les pansements réalisés à partir de la masse adhésive hydrocolloïde selon l'invention peuvent se présenter sous une forme géométrique quelconque, par exemple carrée, rectangulaire, circulaire ou ovale. De même leur taille peut être quelconque et sera adaptée en fonction de la surface de la partie à traiter ou protéger. Par exemple, un pansement destiné au traitement de l'ampoule se présentera sous une forme rectangulaire d'environ 7 cm de longueur et 4 cm de largeur tandis qu'un pansement destiné au traitement de l'ulcère se présentera sous une forme carrée de 10 cm de coté.

De façon pratique, la surface de la masse adhésive hydrocolloïde portant les agglomérats pourra être recouverte d'un film ou pellicule de protection destiné à être retiré, par exemple par pelage, avant la mise en place du pansement sur la plaie et/ou sur la peau.

L'ensemble ainsi formé pourra être lui-même emballé dans une protection étanche formée par exemple au moyen de complexes polyéthylène-aluminium ou dans des blisters.

L'invention sera illustrée par les exemples non limitatifs suivants.

## EXEMPLE 1: Préparation d'un pansement hydrocolloïde standard

On a préparé une masse adhésive hydrocolloïde constituée des composés suivants (quantité exprimée en poids pour 100 grammes de masse).

| N° | Composés | Quantité (en poids pour 100 grammes) |
|---|---|---|
| 1 | Elastomère<br>Copolymère bloc Styrène-Isoprène-Styrène/ Styrène Isoprène (commercialisé sous la dénomination Vector® 4114A par Dexco Polymers LP) | 14,144 |
| 2 | Agent améliorant la cohésion<br>Polyisobutène (commercialisé sous la dénomination Oppanol® B12SFN par BASF) | 3,536 |
| 3 | Plastifiant<br>Huile minérale (commercialisé sous la dénomination Ondina® 933 par Shell) | 12,376 |
| 4 | Tensioactif<br>Copolymère ester acrylique réticulable UV (commercialisé sous la dénomination AcResin® A258UV par BASF) | 6,542 |
| 5 | Résine tackifiante<br>Résine synthétique d'hydrocarbures (commercialisé sous la dénomination de Wingtack® 86 par Safic Alcan) | 26,521 |
| 6 | Hydrocolloïde<br>Carboxyméthylcellulose (commercialisé sous la dénomination CMC Blanose® 7H4XF par Hercules) | 35,714 |
| 7 | Tensioactif<br>Polysorbate 80 (commercialisé sous la dénomination Montanox® 80 par la société SEPPIC) | 0,460 |
| 8 | Antioxydant<br>Dibutyldithiocarbamate de Zinc (commercialisé sous la dénomination Perkacit® ZDBC par la société FLEXSYS (distributeur ARNAUD)) | 0,354 |
| 9 | Antioxydant<br>Pentaérythritol Tetrakis (3-3,5-di-tert-butyl-4-hydroxyphényl)proprionate) (commercialisé sous la dénomination Irganox® 1010 par Ciba Speciality Chemicals) | 0,354 |

Cette masse adhésive hydrocolloïde a été préparée par la mise en oeuvre du procédé suivant :

Dans un malaxeur à bras en Z à une température de consigne de 140°C, on a introduit les composés 1, 2, 3, 8 et 9.

A la 30ème minute, on a ajouté le composé 4.

A la 40ème minute, on a ajouté le composé 7.

A la 45ème minute, on a ajouté le composé 5.

A la 60ème minute, on a ajouté le composé 6.

La vidange du malaxeur a été effectuée à la 80ème minute.

La masse hydrocolloïde adhésive a été chauffée à une température de l'ordre de 110 à 130°C, puis enduite sur un support constitué d'un film en polyuréthane ayant une épaisseur de 30 $\mu$m. Le complexe formé de la masse hydrocolloïde et du support a ensuite été découpé en pansements individuels, couverts, au niveau de la surface hydrocolloïde adhésive libre, d'ailettes de protection en polyester siliconé.

## EXEMPLE 2 : Préparation d'unpansement selon l'invention

a) Préparation d'agglomérats à base de maltodextrine

On a préparé des agglomérats à base de maltodextrine en pulvérisant une solution aqueuse sur une poudre de maltodextrine dans un granulateur à lit d'air fluidisé.

Après séchage à 80°C pendant la durée d'incorporation par spray, on a obtenu des agglomérats ayant une structure générale de type "framboise" présentant une dimension moyenne de 400 $\mu$m environ.

b) Dépôt d'agglomérats à la surface d'une masse adhésive hydrocolloïde

La masse hydrocolloïde adhésive préparée à l'exemple 1 a été chauffée à une température de l'ordre de 110 à 130°C, puis enduite sur un support constitué d'un film en polyuréthane ayant une épaisseur de 30 μm. Le complexe formé par la masse adhésive hydrocolloïde et le film en polyuréthane a été découpé en pansements individuels.

On a déposé ensuite sur la surface libre (non liée au support) de la masse adhésive hydrocolloïde des agglomérats à base de maltodextrine.

Ces agglomérats ont été déposés en une quantité comprise entre 100 à 150 g/m$^2$.

Ces agglomérats ont été déposés au centre du pansement en recouvrant sensiblement 15 % de la surface libre de la masse adhésive.

La surface de la masse hydrocolloïde adhésive portant les agglomérats a été couverte d'ailettes de protection en polyester siliconé.

### EXEMPLE 3 : Mise en évidence des propriétés du pansement conforme à l'invention

On a mesuré l'adhésivité des pansements tel que préparés aux exemples 1 et 2, après contact avec du sérum physiologique pour simuler l'exsudation d'une plaie, afin d'évaluer la différence de comportement au retrait entre un pansement comprenant une masse adhésive hydrocolloïde traditionnelle (exemple 1) et un pansement selon l'invention comprenant une masse adhésive hydrocolloïde dont la surface destinée à venir au contact de la peau porte des agglomérats (exemple 2).

A cet effet, on a utilisé la méthode dite du "tack à la sonde" qui vise à mesurer le décollement d'une sonde cylindrique appliquée sur un adhésif avec une contrainte de pression $P_0$ pendant un temps t donné dans des conditions de température et d'humidité prédéterminées.

Appareillage

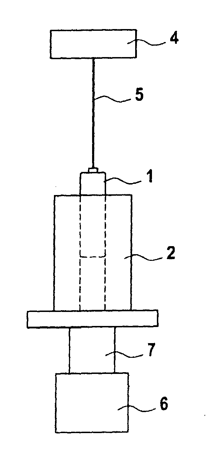

Plus précisément, cette méthode a été mise en oeuvre au moyen de l'appareil représenté à la figure 1.

Cet appareil est essentiellement constitué :

- d'une sonde cylindrique 1 présentant une extrémité finement polie susceptible de se déplacer longitudinalement (de bas en haut dans l'exemple représenté) au travers d'un guide sonde 2 constitué d'un cylindre percé en son centre d'un trou libre permettant le libre passage sans frottement de la sonde ;
- d'un dynamomètre électronique comprenant une mâchoire mobile 4 pouvant être reliée à la sonde 1 par une chaînette 5 et un socle 6 sur lequel peut être emboîté un plateau 7 ; ledit dynamomètre étant en outre relié à un système d'acquisition et d'enregistrement des données telles qu'en particulier la vitesse de descente de la sonde, la pression d'accostage de la sonde sur l'adhésif, le temps de contact entre la sonde et l'adhésif et la vitesse de remontée de la sonde.

Echantillons

Pansement standard

En suivant le protocole expérimental de l'exemple 1, on a préparé un pansement sensiblement carré de 20 cm de côté.

Ce pansement, a été recouvert d'un ruban adhésif double face sur la face formant support. Un rouleau presseur a été employé afin de bien faire adhérer ledit ruban à la surface du pansement.

Des échantillons circulaires de même surface que la sonde ont été découpés dans l'ensemble ainsi constitué par le pansement et le ruban adhésif précité à l'aide d'un emporte pièce circulaire de 30 mm de diamètre.

Pansement selon l'invention

Un second jeu d'échantillons de même nature et de même dimension a été préparé.

On a déposé des agglomérats à base de maltodextrine tels que ceux préparés à l'exemple 2 sur la surface de ces échantillons (les agglomérats en excès ayant été éliminés afin de recouvrir uniformément la surface de l'échantillon).

Mode opératoire :

- On a emboîté le plateau 7 sur le socle 6 du dynamomètre.
- On a fixé la chaînette 5 de la sonde dans la mâchoire mobile 4 du dynamomètre.
- On a nettoyé la sonde 1 avec un solvant (cette opération étant répétée avant chaque mesure).
- On a appliqué un échantillon (coté double face) tel que préparé précédemment sur la sonde.
- On a déposé 50 $\mu$l de sérum physiologique sur le plateau 7 au milieu de la zone délimitée par le guide-sonde.
- On a programmé les paramètres du dynamomètre (vitesse de descente, pression d'accostage, temps de contact et vitesse de remontée).
- On a procédé à la mesure en effectuant les opérations suivantes: descente de la sonde, mise en contact de l'échantillon adhésif et du sérum physiologique.
- On a enregistré la force de décollement en kPa que l'on a traduite en contrainte d'arrachement C suivant la relation :

$$C = F/S$$

où F = Force exprimée en N et S= Surface exprimée en m$^2$.

Paramètres du test:

Contrainte d'application : 1.7kPa (sonde aluminium de 121 g et diamètre de 30 mm)
Vitesse d'essai : 300 mm/min
Rouleau presseur : 4 kg
Temps de contact : 1 s

Les mesures ont été réalisées sur 3 échantillons de chacun des pansements standard et selon l'invention et répétées 6 fois.

Résultats :

Les résultats des mesures ainsi effectuées exprimés en kPa ont été reportés dans le tableau I.

TABLEAU I

| Essai N° | Exemple 1 | Exemple 2 |
|----------|-----------|-----------|
| 1 | 138,8 | 30,1 |
| 2 | 182,8 | 25,8 |
| 3 | 171,2 | 27,7 |
| 4 | 160,7 | 34,5 |
| 5 | 134,4 | 21,7 |
| 6 | 194 | 27,7 |

Observations du comportement des pansements selon les exemples 1 et 2 en présence de sérum physiologique.

| Conditions | Observations après contact avec sérum physiologique |
|------------|------------------------------------------------------|
| Exemple 1 + 50$\mu$l sérum physiologique | Gélification totale après 15 minutes |
| Exemple 2 +50$\mu$l sérum physiologique | Solubilisation en une seconde |

Conclusions

La solubilisation quasi totale des agglomérats est très rapide (1s).

Le test du "tack à la sonde" a permis de constater que l'adhésivité au retrait d'un pansement adhésif hydrocolloïde selon l'invention est et bien inférieure à celle d'un pansement standard sans agglomérats.

Lorsque la masse adhésive hydrocolloïde du pansement porte des agglomérats, l'adhésivité au retrait de celui-ci devient minime lorsqu'il rentre en contact avec du sérum physiologique, même en très faible quantité.

Ainsi, un pansement selon l'invention, c'est à dire comprenant une masse adhésive hydrocolloïde portant des agglomérats de particules hydrosolubles ou gélifiables au niveau de sa surface destinée à entrer en contact avec la plaie, pourra être facilement repositionné après une première application, et ce dans les secondes qui suivent ladite première application.

## Revendications

1. Pansement comprenant une masse adhésive comprenant des hydrocolloïdes **caractérisé en ce qu'**il comprend, sur une partie au moins de la surface de la masse adhésive destinée à venir au contact de la plaie en position d'utilisation, une pluralité d'agglomérats constitués de particules liées entre elles, de préférence selon une configuration multicouches, lesdites particules étant constituées pour 90 % au moins, et de préférence 95 % au moins d'entre elles d'un (ou plusieurs) matériau(x) :

   - choisi(s) parmi

      - les polysaccharides et en particulier l'amidon, les amidons modifiés, la maltodextrine, les gommes comme en particulier la gomme arabique, la gomme xanthane ou la gomme d'acacia, le pullulan, les dextranes, la cellulose, les dérivés cellulosiques comme en particulier la méthylcellulose, l'hydroxypropylméthylcellulose, l'hydroxypropylcellulose, l'hydroxyéthylcellulose, les sucres, la pectine, les alginates, les carraghénanes, l'agar-agar ;
      - les protéines et en particulier la gélatine, l'albumine, le collagène et la caséine ;
      - les polymères synthétiques superabsorbants et en particulier les polyacrylates ;
      - les polymères synthétiques hydrosolubles et en particulier l'alcool polyvinylique ;

   et les mélanges de ces composés ; et
   - apte(s) à se solubiliser ou gélifier en moins de 10 secondes et de préférence encore en moins de 1 seconde au contact des exsudats de la plaie.

2. Pansement selon la revendication 1, **caractérisé en ce que** les particules précitées ont une dimension moyenne de 500 nm à 1000 $\mu$m de préférence 1 $\mu$m à 500 $\mu$m

3. Pansement selon l'une des revendications précédentes, **caractérisé en ce que** les agglomérats précités ont une dimension moyenne de 5 à 2 000 $\mu$m.

4. Pansement selon l'une des revendications précédentes, caractérisé en ce les agglomérats précités sont présents en une quantité de 5 à 500 g par m$^2$, et de préférence de 10 à 150 g par m$^2$ de pansement.

5. Pansement selon l'une des revendications précédentes, **caractérisé en ce que** les agglomérats précités recouvrent 10 à 60 % de la surface de la masse adhésive destinée à venir au contact de la plaie en position d'utilisation.

6. Pansement selon l'une des revendications précédentes, **caractérisé en ce que** le matériau constituant les particules précité est la maltodextrine.

7. Pansement selon l'une des revendications précédentes, **caractérisé en ce que** les agglomérats contiennent une ou plusieurs substance(s)active(s) choisie(s) parmi les accélérateurs de cicatrisation, les antifongiques, les antimicrobiens ou antibactériens, les agents hydratants, les anesthésiques locaux, les anti-inflammatoires ou un mélange de ces composés.

8. Pansement selon la revendication 7, **caractérisé en ce que** la substance active précitée est l'acide hyaluronique.

9. Pansement selon l'une des revendications précédentes, **caractérisé en ce qu'**il comprend un support.

10. Pansement selon l'une des revendications précédentes, **caractérisé en ce qu'**il comprend une couche absorbante

située entre le support et la masse hydrocolloïde.

**Patentansprüche**

1.  Verband, umfassend eine Hydrokolloide umfassende haftende Masse, **dadurch gekennzeichnet, dass** er auf wenigstens einem Teil der Fläche der haftenden Masse, die dazu bestimmt ist, in Gebrauchsposition mit der Wunde in Kontakt zu kommen, eine Vielzahl von Agglomeraten, welche von untereinander verbundenen Partikeln gebildet sind, vorzugsweise in einer Mehrlagenanordnung, umfasst, wobei die Partikel zu wenigstens 90 % und vorzugsweise wenigstens 95 % unter sich von einem (oder mehreren) Material(ien) gebildet sind:

    - das (die) ausgewählt ist (sind) aus

       - den Polysacchariden und insbesondere Stärke, den modifizierten Stärken, Maltodextrin, Gummis, wie insbesondere Gummi arabicum, Xanthangummi oder Akaziengummi, Pullulan, den Dextranen, Cellulose, den Cellulosederivaten, wie insbesondere Methylcellulose, Hydroxypropylmethylcellulose, Hydroxypropylcellulose, Hydroxyethylcellulose, den Zuckern, Pektin, den Alginaten, Carrageen, Agar-Agar;
       - den Proteinen und insbesondere Gelatine, Albumin, Kollagen und Kasein;
       - den synthetischen superabsorbierenden Polymeren und insbesondere den Polyacrylaten;
       - den synthetischen wasserlöslichen Polymeren und insbesondere Polyvinylalkohol;

    sowie den Mischungen dieser Verbindungen; und
    - das (die) geeignet ist (sind), in weniger als 10 Sekunden und vorzugsweise weiterhin in weniger als 1 Sekunde bei Kontakt mit den Exsudaten der Wunde löslich zu werden oder zu gelieren.

2.  Verband nach Anspruch 1, **dadurch gekennzeichnet, dass** die vorgenannten Partikel eine mittlere Abmessung von 500 nm bis 1000 $\mu$m, vorzugsweise 1 $\mu$m bis 500 $\mu$m haben.

3.  Verband nach einem der vorhergehenden Ansprüche, **dadurch gekennzeichnet, dass** die vorgenannten Agglomerate eine mittlere Abmessung von 5 bis 2000 $\mu$m haben.

4.  Verband nach einem der vorhergehenden Ansprüche, **dadurch gekennzeichnet, dass** die vorgenannten Agglomerate in einer Menge von 5 bis 500 g pro m$^2$ und vorzugsweise von 10 bis 150 g pro m$^2$ Verband vorhanden sind.

5.  Verband nach einem der vorhergehenden Ansprüche, **dadurch gekennzeichnet, dass** die vorgenannten Agglomerate 10 bis 60 % der Fläche der haftenden Masse, die dazu bestimmt ist, in Gebrauchsposition mit der Wunde in Kontakt zu kommen, bedecken.

6.  Verband nach einem der vorhergehenden Ansprüche, **dadurch gekennzeichnet, dass** das vorgenannte Material, das die Partikel bildet, Maltodextrin ist.

7.  Verband nach einem der vorhergehenden Ansprüche, **dadurch gekennzeichnet, dass** die Agglomerate einen oder mehrere Wirkstoff(e) enthalten, der (die) aus den Wundheilungsbeschleunigern, den Fungiziden, den antimikrobiellen Wirkstoffen oder antibakteriellen Wirkstoffen, den feuchtigkeitsspendenden Mitteln, den Lokalanästhetika, den entzündungshemmenden Mitteln oder einer Mischung dieser Verbindungen ausgewählt ist (sind).

8.  Verband nach Anspruch 7, **dadurch gekennzeichnet, dass** der vorgenannte Wirkstoff Hyaluronsäure ist.

9.  Verband nach einem der vorhergehenden Ansprüche, **dadurch gekennzeichnet, dass** er einen Träger umfasst.

10. Verband nach einem der vorhergehenden Ansprüche, **dadurch gekennzeichnet, dass** er eine zwischen dem Träger und der Hydrokolloidmasse befindliche absorbierende Schicht umfasst.

**Claims**

1.  A wound dressing comprising an adhesive mass comprising hydrocolloids, **characterized in that** the dressing comprises, on a portion at least of the surface of the adhesive mass intended to come into contact with the wound in the position of use, a plurality of agglomerates composed of particles bonded to one another, preferably according to a multilayer configuration, said particles being composed, for 90% at least and preferably 95% at least of them, of one (or more) material(s);

- chosen from

- polysaccharides and in particular starch, modified starches, maltodextrin, gums, such as, in particular, gum arabic, xanthan gum or acacia gum, pullulan, dextrans, cellulose, cellulose derivatives, such as, in particular, methylcellulose, hydroxypropylmethylcellulose, hydroxypropylcellulose or hydroxyethylcellulose, sugars, pectin, alginates, carragheenans or agar;
- proteins, in particular gelatin, albumin, collagen and casein;
- superabsorbent synthetic polymers, in particular polyacrylates;
- water-soluble synthetic polymers, in particular polyvinyl alcohol;

and the mixtures of these compounds; and
- capable of dissolving or gelling in less than 10 seconds and more preferably in less than 1 second on contact with the exudates from the wound.

2. The dressing as claimed in claim 1, **characterized in that** the abovementioned particles have a mean size of 500 nm to 1000 $\mu$m, preferably of 1 $\mu$m to 500 $\mu$m.

3. The dressing as claimed in either of the preceding claims, **characterized in that** the abovementioned agglomerates have a mean size of 5 to 2000 $\mu$m.

4. The dressing as claimed in one of the preceding claims, **characterized in that** the abovementioned agglomerates are present in an amount of 5 to 500 g per m$^2$ and preferably of 10 to 150 g per m$^2$ of dressing.

5. The dressing as claimed in one of the preceding claims, **characterized in that** the abovementioned agglomerates cover from 10 to 60% of the surface of the adhesive mass intended to come into contact with the wound in the position of use.

6. The dressing as claimed in one of the preceding claims, **characterized in that** the abovementioned material constituting the particles is maltodextrin.

7. The dressing as claimed in one of the preceding claims, **characterized in that** the agglomerates comprise one or more active substance(s) chosen from healing accelerators, antifungals, antimicrobials or antibacterials, moisturizing agents, local anesthetics, anti-inflammatories or a mixture of these compounds.

8. The dressing as claimed in claim 7, **characterized in that** the abovementioned active substance is hyaluronic acid.

9. The dressing as claimed in one of the preceding claims, **characterized in that** it comprises a backing.

10. The dressing as claimed in one of the preceding claims, **characterized in that** it comprises an absorbent layer situated between the backing and the hydrocolloid mass.

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 264299 A
- EP 503029 A
- EP 1020198 A
- FR 2495473
- EP 0621042 A
- EP 1061965 A
- EP 1165717 A
- EP 0927051 A
- FR 2392076
- WO 9810801 A

**Littérature non-brevet citée dans la description**

- **DONATAS SATAS.** Handbook of Pressure Sensitive Technology. 1999, 346-398